# EUROPEAN PATENT APPLICATION

(11) **EP 1 744 175 A2**
(43) Date of publication of application: **17.01.2007**
(21) Application number: 06014450.8
(22) Date of filing: 12.07.2006
(51) Int. Cl.: G01S 7/52, A61B 8/00

(54) **Ultrasound diagnostic system and method of forming Tx and Rx beams by using delay data**

(30) Priority: 15.07.2005 KR 20050064419
(71) Applicant: MEDISON CO., LTD., Kangwon-do 250-870 (KR)
(72) Inventor: Kye, Sang Bum, Gangnam-gu, 135-280 Soul (KR)
(74) Representative: Schmid, Wolfgang

(57) **Abstract**

An ultrasound diagnostic system including: a memory unit for storing transmit (Tx) delay data and receive (Rx) delay data necessary for forming a Tx beam and a Rx beam; a Tx beam forming unit for adjusting delays of Tx signals by referencing the Tx delay data stored in the memory unit to form the Tx beam; a probe for converting the Tx signals into ultrasound signals and transmitting the ultrasound signals into a target object, the probe being configured to receive ultrasound echo signals reflected from the target object to output electrical Rx signals; and a Rx beam forming unit for adjusting delays of the Rx signals by referencing the Rx delay data stored in the memory unit to form the Rx beam.

## Description

The present invention generally relates to an ultrasound diagnostic system, and more particularly to a method for accurately forming a transmit beam and a receive beam by using delay data stored in a memory so as to obtain a high quality ultrasound image in an ultrasound diagnostic system.

An ultrasound diagnostic system has become an important and popular diagnostic tool since it has a wide range of applications. Specifically, due to its non-invasive and non-destructive nature, the ultrasound diagnostic system has been extensively used in the medical profession. Modem high-performance ultrasound diagnostic systems and techniques are commonly used to produce two or three-dimensional diagnostic images of internal features of an object (e.g., human organs).

The ultrasound diagnostic system generally uses a wide bandwidth transducer to transmit and receive ultrasound signals. The ultrasound diagnostic system forms images of human internal tissues by electrically exciting an acoustic transducer element or an array of acoustic transducer elements to generate ultrasound signals that travel into the body. The ultrasound signals produce ultrasound echo signals since they are reflected from body tissues, which appear as discontinuities to the propagating ultrasound signals. Various ultrasound echo signals return to the transducer element and are converted into electrical signals, which are amplified and processed to produce ultrasound data for an image of the tissues. The ultrasound diagnostic system is very important in the medical field since it provides physicians with real-time and high-resolution images of human internal features without the need for invasive observation techniques such as surgery.

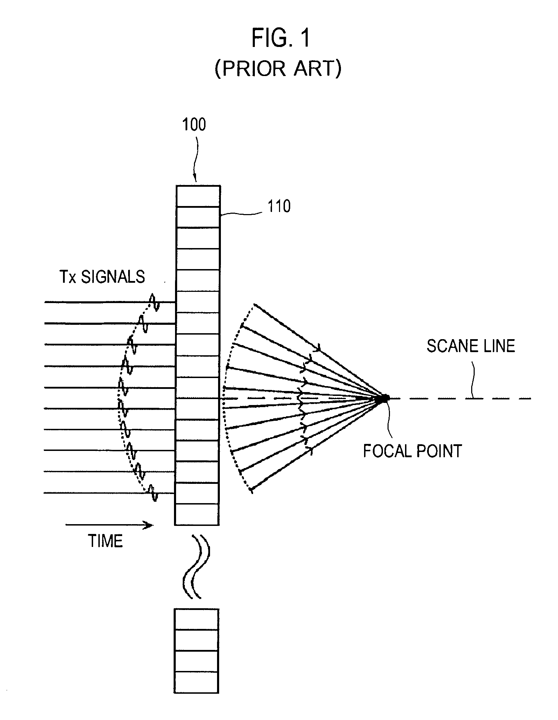

In the ultrasound diagnostic system, a beam former is one of the important components for obtaining a high-resolution ultrasound image. Recently, a digital beam forming technology, which is capable of focusing multiple points, is applied to the beam former. Fig. 1 shows a pattern of transmit (Tx) signals for forming a Tx beam, which is to be focused on a focal point. As shown in Fig. 1, the Tx signals are applied to a transducer array 100 included in a probe, thereby generating ultrasound signals. The transducer array 100 consists of a plurality of transducer elements 110. Each of the Tx signals is converted into the ultrasound signal by each transducer element 110.

Generally, a Tx beam focusing the ultrasound signals outputted from the transducer elements on focal points set along a scan line in a target object is used to improve the resolution of an ultrasound image. When the transducer elements are configured in a linear array, the time of arrival for each Tx signal at each transducer element should be controlled to focus the ultrasound signals on the focal point. That is, each Tx signal should be appropriately delayed such that the Tx signal arrives first at a transducer element, which is farthest from the focal point, while arriving last to a transducer element that is closest to the focal point. This is so that the ultrasound signals, which are outputted from each transducer element, can arrive at the focal point at the same time.

Ultrasound echo signals, which are reflected from the focal point, are transmitted to the transducer elements. The ultrasound echo signals are converted into electrical receive signals (hereinafter referred to as Rx signals) by the transducer elements. The ultrasound echo signals reflected from the focal point of the target object are received by each transducer element at different times according to the position of each transducer element in the transducer array. Therefore, the delays of the Rx signals must be adjusted to form a receive beam. Adjusting the delays of the Tx and Rx signals is carried out by the beam former in the ultrasound diagnostic system.

The diagnosis of a target object becomes more accurate as the quality of an ultrasound image increases. There are many factors for enhancing the quality of the ultrasound images. Especially, a Tx/Rx beam forming process is one of the important factors for obtaining a high quality ultrasound image. The conventional Tx/Rx beam forming process is carried out based on an algorithm reflecting the delays of the Tx and Rx signals under the condition that the transducer elements are in direct contact with the surface of a target object. However, there are various materials between the transducer elements and the surface of the target object such as a matching layer, lens, protective rubber and the like. Therefore, an accurate Tx/Rx beam cannot be formed when the conventional algorithm, which ignores such materials, is adopted.

The present invention provides a method of accurately forming a Tx beam and a Rx beam by using delays previously calculated by considering materials existing between transducer elements and a surface of a target object.

According to one aspect of the present invention, there is provided an ultrasound diagnostic system including: a memory unit for storing transmit (Tx) delay data and receive (Rx) delay data necessary for forming a Tx beam and a Rx beam; a Tx beam forming unit for adjusting delays of Tx signals by referencing the Tx delay data stored in the memory unit to form the Tx beam; a probe for converting the Tx signals into ultrasound signals and transmitting the ultrasound signals into a target object, the probe being configured to receive ultrasound echo signals reflected from the target object to output electrical Rx signals; and a Rx beam forming unit for adjusting delays of the Rx signals by referencing the Rx delay data stored in the memory unit to form the Rx beam.

According to another aspect of the present invention, there is provided a method of forming a transmit (Tx) beam and a receive (Rx) beam in an ultrasound diagnostic system, the method including the steps of: storing transmit (Tx) delay data and receive (Rx) delay data necessary for forming a Tx beam and a Rx beam; adjusting delays of Tx signals by referencing the stored Tx delay data to form the Tx beam; converting the Tx signals into ultrasound signals with transducer elements and transmitting the ultrasound signals into a target object; converting ultrasound echo signals, which are received by the transducer elements from the target object, into electrical Rx signals; and adjusting delays of the Rx signals by referencing the stored Rx delay data to form the Rx beam.

The above and other objects and features of the present invention will become apparent from the following description of preferred embodiments given in conjunction with the accompanying drawings, in which:
Fig. 1 is a schematic diagram showing a pattern of Tx signals for forming a Tx beam;
Fig. 2 is a block diagram showing an ultrasound diagnostic system constructed in accordance with the present invention;
Fig. 3 is a diagram schematically showing a structure of a probe;
Fig. 4 is a schematic diagram showing that an ultrasound echo signal from a focal point arrives at each transducer element at different times;
Fig. 5 is a graph showing the relationship between a delay of an Rx signal and a time; and
Fig. 6 is a flow chart showing a process for forming an ultrasound image in an ultrasound diagnostic system constructed in accordance with the present invention.

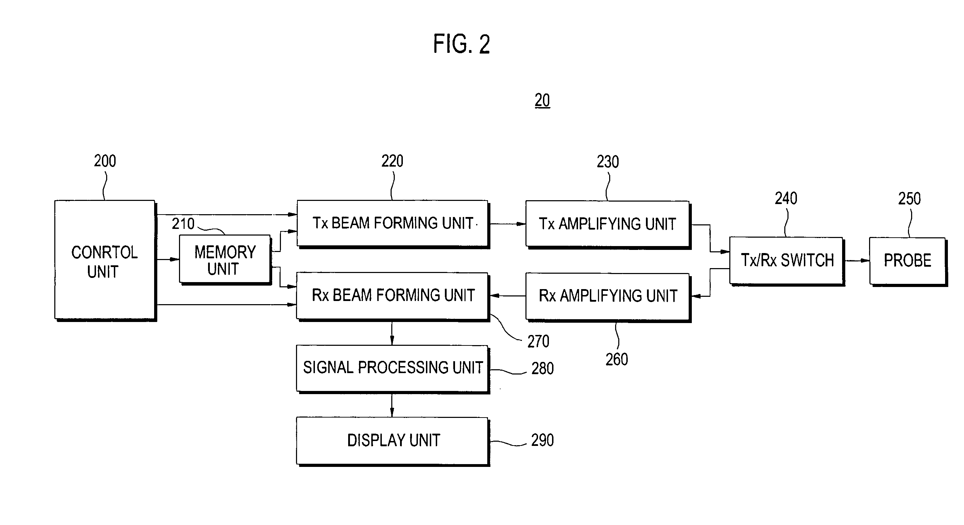

Fig. 2 is a block diagram illustrating an ultrasound diagnostic system constructed in accordance with the present invention. As shown in Fig. 2, the ultrasound diagnostic system 20 includes a control unit 200, a memory unit 210, a Tx beam forming unit 220, a Tx amplifying unit 230, a Tx/Rx switch 240, a probe 250, a Rx amplifying unit 260, a Rx beam forming unit 270, a signal processing unit 280 and a display unit 290.

The Tx beam forming unit 220 and the Rx beam forming unit 270 adjust the delays of Tx signals and Rx signals to form a Tx beam (from Tx signals) and a Rx beam (from Rx signals), respectively, under the control of the control unit 200. The Tx signals are signals, which are transmitted into the probe 250 to generate ultrasound signals. The Rx signals are electrical signals outputted from the transducer elements included in the probe 250 in response to ultrasound echo signals.

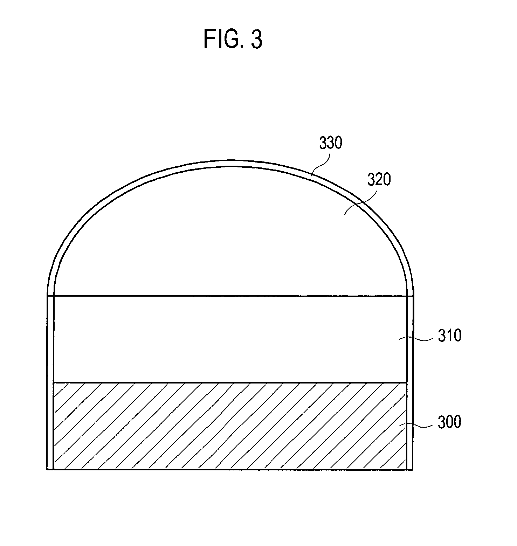

The memory unit 210 stores the data related to the Tx and Rx delays. The Tx delays represent the delays of the respective Tx signals, which are necessary for forming the Tx beam focused on a focal point on a scan line in the target object. The Tx delays are previously calculated for all the focal points, which may be set in the target object by considering the propagation speeds of an ultrasound signal in materials existing between the transducer elements and the surface of the target object. The Tx delay may be calculated differently and stored according to the types of the target object. As shown in Fig. 3, the materials, which exist between the transducer elements 300 and the surface of the target object, may include a matching layer 310, a lens 320 and a protective rubber 330 as a cover of the probe 250. Also, the memory unit 210 stores the data relating to the Rx delays of the respective Rx signals for forming the Rx beam. The Rx delays are caused due to the different distances between each transducer element and the focal point. The Rx delays are previously calculated for all focal points, which may be set in the target object by considering the propagation speeds of an ultrasound signal in materials existing between the transducer elements and the surface of the target object.

The Tx beam forming unit 220 adjusts the pattern of the Tx signals by referencing the Tx delay data stored in the memory unit 210 when forming the Tx beam. The Tx amplifying unit 230 amplifies the Tx signals, which are transferred from the Tx beam forming unit 220. The amplified Tx signals are transmitted to the probe 250 through the Tx/Rx switch 240. The Tx signals are converted into the ultrasound signals by the transducer elements included in the probe 250, wherein the ultrasound signals are transmitted into the target object.

The ultrasound echo signals reflected from the focal point are converted to electrical Rx signals. Then, the Rx signals are transmitted to the Rx beam forming unit 270 through the Tx/Rx switch 240 and the Rx amplifying unit 260. The Rx beam forming unit 270 adjusts the delays of the Rx signals by referencing the Rx delay data stored in the memory 310 under the control of the control unit 200 when forming the Rx beam. The Rx signals, which adjust the delays thereof, are summed and transmitted to the signal processing unit 280. The ultrasound diagnostic system 20 may include a codec unit (not shown) for compressing and decompressing the Tx and Rx delay data. The codec unit may be implemented by using H.264, which is a digital video codec standard in Moving Picture Experts Group (MPEG). Thereafter, an appropriate signal processing is carried out for the signals outputted from the Rx beam forming unit 270, thereby obtaining the ultrasound image data. The ultrasound image data are transmitted to the display unit 290 so as to be displayed as an ultrasound image of the target object.

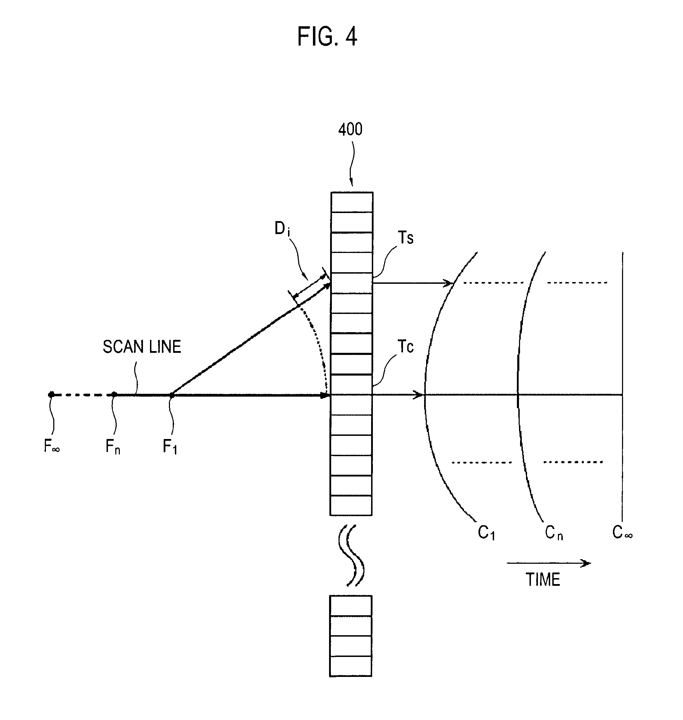

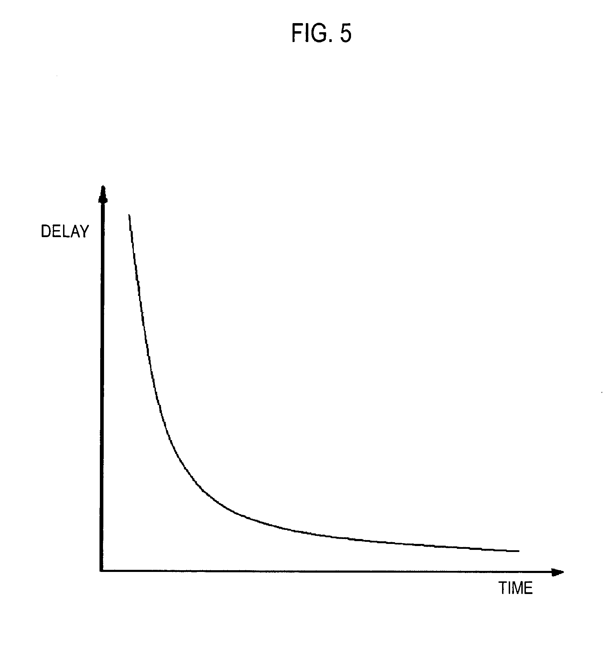

Fig. 4 is a schematic diagram, which shows that an ultrasound echo signal from a focal point reaches each of the transducer elements at different times. As shown in Fig. 4, the ultrasound echo signals reflected from focal points F₁, Fₙ... F_{∞} arrive at each transducer element at different times due to the position of each transducer element for the focal point. For example, an ultrasound echo signal from focal point F₁ arrives at a transducer element Tₛ by being delayed as Dᵢ (compared to an arriving time in which the ultrasound echo signal reflected from focal point F₁ arrives at a transducer element T_{c}). Curves C₁, Cₙ and C_{∞} show the delay pattern of the Rx signals outputted from the transducer elements in response to the ultrasound echo signals reflected from F₁, Fₙ, F_{∞}, respectively. As the focal point becomes more distant from the transducer element, the curve showing the delay pattern of the Rx signal becomes similar to a straight line. That is, the delay of the Rx signal at a transducer element is inversely proportional to the time, as shown in Fig. 5.

The delays of the Rx signals, which are represented as a graph shown in Fig. 5, are calculated for each transducer element by considering the propagation speeds of the ultrasound signal in the materials existing between the transducer element and the surface of the target object. Then, the calculated delays are stored in the memory unit 210. That is, the memory unit 210 stores the data corresponding to the delay graphs of the Rx signals shown in Fig. 5. The delay data may be stored in relation to manufacturing the ultrasound diagnostic system 20 and be updated for operating the ultrasound diagnostic system 20.

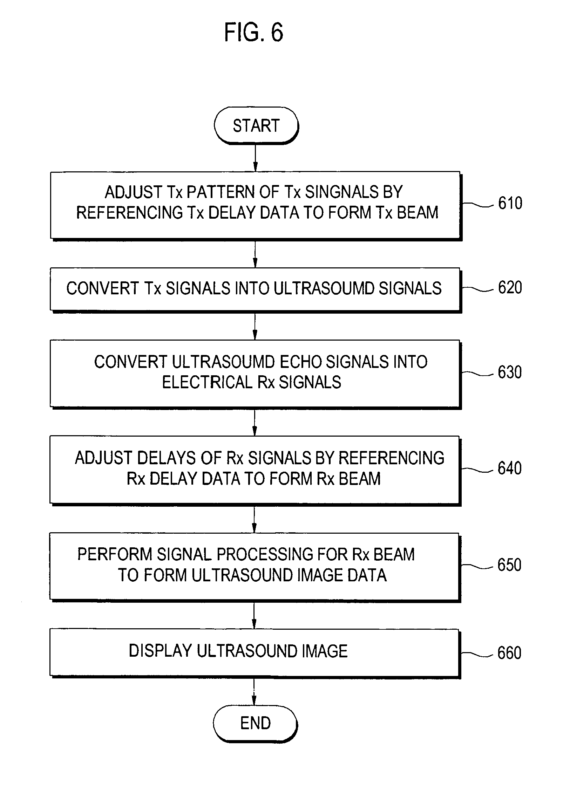

A method of forming the Tx and Rx beams by using the delay data stored in the memory unit 130 will be described below in view of Figs. 2 to 6.

The Tx beam forming unit 220 adjusts the Tx pattern of Tx signals so as to form a desirable Tx beam under the control of the control unit 100 at step S610. The adjustment of the Tx pattern ofTx signals is performed through individually adjusting the delay of each Tx signal by referencing the Tx delay data stored in the memory unit 210. The adjusted Tx signals are transmitted to the transducer elements to generate the ultrasound signals, which are to be transmitted into the target object, at step S620. The transducer elements receive the ultrasound echo signals reflected from the target object and convert the ultrasound echo signals into electrical Rx signals at step S630. The Rx beam forming unit 270 receives the Rx signals and adjusts the delays of the Rx signals by referencing the delay data stored in the memory unit 130 such that the Rx signals have the same phase, thereby forming the Rx beam at step S640. After forming the Rx beam, the signal processing unit 280 performs signal processing for the Rx beam to form the ultrasound image data at step S650. The ultrasound image data are then transmitted to the display unit 290 so as to be displayed with an ultrasound image at step S660.

As mentioned above, since the Tx and Rx beams are formed by considering the materials existing between the transducer elements and the surface of the target object, an improved ultrasound image can be obtained. Also, the ultrasound image data can be obtained without using a specific algorithm for forming the Tx and Rx beams.

While the present invention has been described and illustrated with respect to a preferred embodiment of the invention, it will be apparent to those skilled in the art that variations and modifications are possible without deviating from the broad principles and teachings of the present invention, which should be limited solely by the scope of the claims appended hereto.

## Claims

1. An ultrasound diagnostic system, comprising:
a memory unit for storing transmit (Tx) delay data and receive (Rx) delay data necessary for forming a Tx beam and a Rx beam;
a Tx beam forming unit for adjusting delays of Tx signals by referencing the Tx delay data stored in the memory unit to form the Tx beam;
a probe for converting the Tx signals into ultrasound signals and transmitting the ultrasound signals into a target object, the probe being configured to receive ultrasound echo signals reflected from the target object to output electrical Rx signals; and
a Rx beam forming unit for adjusting delays of the Rx signals by referencing the Rx delay data stored in the memory unit to form the Rx beam.

2. The ultrasound diagnostic system of Claim 1, further comprising:
a control unit for controlling operations of the ultrasound diagnostic system;
a Tx amplifying unit for amplifying the Tx signals outputted from the Tx beam forming unit;
a Rx amplifying unit for amplifying the Rx signals outputted from the probe;
a signal processing unit for performing signal processing for the Rx beam to generate ultrasound image data; and
a display unit for displaying an ultrasound image based on the ultrasound image data.

3. The ultrasound diagnostic system of Claim 1, wherein the Tx delay data and the Rx delay data are calculated in consideration of propagation speeds of the ultrasound signals in a matching layer, a lens and a protective rubber existing between the transducer elements and the surface of the target object.

4. The ultrasound diagnostic system of Claim 1, further comprising a codec unit for compressing the Tx delay data and the Rx delay data to be stored in the memory unit and decompressing the compressed Tx delay data and Rx delay data.

5. A method of forming a transmit (Tx) beam and a receive (Rx) beam in an ultrasound diagnostic system, comprising the steps of:
storing transmit (Tx) delay data and receive (Rx) delay data necessary for forming a Tx beam and a Rx beam;
adjusting delays of Tx signals by referencing the stored Tx delay data to form the Tx beam;
converting the Tx signals into ultrasound signals and transmitting the ultrasound signals into a target object;
converting ultrasound echo signals received from the target object into electrical Rx signals; and
adjusting delays of the Rx signals by referencing the stored Rx delay data to form the Rx beam.

6. The method of Claim 5, further comprising the steps of:
amplifying the Tx signals adjusting the delay thereof; and
amplifying the Rx signals converted from the ultrasound echo signals.

7. The method of Claim 6, wherein the Tx delay data and the Rx delay data are calculated in consideration of propagation speeds of the ultrasound signals in a matching layer, a lens and a protective rubber existing between the transducer elements and a surface of the target object.

8. The method of Claim 5, further comprising the step of compressing the Tx delay data and the Rx delay data before the storing step.
